# EUROPÄISCHE PATENTANMELDUNG

(11) **EP 2 153 787 A1**
(43) Veröffentlichungstag der Anmeldung: **17.02.2010**
(21) Anmeldenummer: 09008864.2
(22) Anmeldetag: 07.07.2009
(51) Int. Cl.: A61B 17/80

(54) **Platte zur Fixierung von Knochenfragmenten**

(30) Priorität: 11.08.2008 DE 102008037204
(71) Anmelder: Buck, Alfred Ernst, 71149 Bondorf (DE)
(72) Erfinder: Buck, Alfred Ernst, 71149 Bondorf (DE)
(74) Vertreter: Herden, Andreas F.

(57) **Zusammenfassung**

Die Erfindung betrifft eine Platte zur Fixierung von Knochenfragmenten, wobei die Platte aus einem gepressten Formkörper aus Draht besteht, vorzugsweise einem gepressten Gestrick.

## Beschreibung

### Gebiet der Erfindung

Die Erfindung betrifft eine Platte zur Fixierung von Knochenfragmenten sowie ein Verfahren zur Herstellung einer Platte zur Fixierung von Knochenfragmenten.

### Hintergrund der Erfindung

Platten zur Fixierung von Knochenfragmenten, auch als Knochenplatten bezeichnet, sind bekannt. Es handelt sich dabei in der Regel um massive Platten, in welchen Bohrungen angebracht sind, um Knochenstücke mittels Schrauben oder Drähten zu fixieren. Derartige Platten sind je nach Einsatzzweck in unterschiedlichsten Formen verfügbar.

Bekannte Knochenplatten bestehen üblicherweise aus Metall, insbesondere aus chirurgischem Edelstahl oder Titan. Zur sicheren Fixierung der Knochenfragmente, insbesondere wenn es sich um größere Fragmente wie Femur-Fragmente handelt, ist in der Regel eine hohe Festigkeit der Knochenplatte erforderlich. Knochenplatten aus Metall haben in der Regel eine hinreichend hohe Festigkeit. Die hohe Festigkeit geht aber mit einer sehr hohen Steifigkeit der Knochenplatte einher, so dass es in der Regel nicht oder nur kaum möglich ist, eine Knochenplatte während der jeweiligen Operation derart zu verformen, dass diese sich genau den Krümmungen der Knochenfragmente anpasst. Zum Ausgleich von Toleranzen verfügen Knochenplatten häufig über Langlöcher, so dass ein gewisses Spiel zwischen der Befestigungsschraube und der Knochenplatte vorhanden ist. Ein derartiges Spiel ist naturgemäß nachteilig für den Heilungsverlauf.

Des Weiteren kann es während des Heilungsprozesses zu Bewegungen der Knochenfragmente gegeneinander kommen. Insbesondere geht eine Frakturheilung und ein dabei entstehender Knochenwulst häufig mit einem leichten Auseinanderschieben der Knochenfragmente einher. Hierbei können herkömmliche Knochenplatten nicht nachgeben, was zum Einen nachteilig für den Heilungsverlauf sein kann. Zum Anderen kann es zu Verspannungen kommen, wodurch Schrauben, mit welchen die Platte fixiert ist, derart fest sitzen, dass diese zu einem späteren Zeitpunkt kaum noch entfernt werden können.

### Aufgabe der Erfindung

Der Erfindung liegt demgegenüber die Aufgabe zugrunde, die genannten Nachteile bekannter Knochenplatten zumindest zu reduzieren.

Insbesondere ist es eine Aufgabe der Erfindung, eine Knochenplatte bereitzustellen, welche einfach einzusetzen ist und sich Krümmungen der Knochenfragmente bis zu einem gewissen Maß anpasst.

Eine weitere Aufgabe der Erfindung ist es, eine Knochenplatte bereitzustellen, welche eine hohe Stabilität aufweist, gleichzeitig aber geringen Verschiebungen der Knochenfragmente zueinander, etwa bei deren Zusammenwachsen, nachgibt.

Eine weitere Aufgabe der Erfindung ist es, eine stabile Knochenplatte mit einem geringen Gewicht bereitstellen zu können.

### Zusammenfassung der Erfindung

Die Aufgabe der Erfindung wird bereits durch eine Platte zur Fixierung von Knochenfragmenten sowie durch ein Verfahren zur Herstellung einer Platte zur Fixierung von Knochenfragmenten nach einem der unabhängigen Ansprüche gelöst.

Bevorzugte Ausführungsformen und Weiterbildungen der Erfindung sind den jeweiligen Unteransprüchen zu entnehmen.

Die Erfindung betrifft des Weiteren ein Implantat aus einem gepressten Formkörper sowie ein Implantat, welches bioresorbierbar ausgebildet ist.

Der Erfinder hat herausgefunden, dass sich gepresste Formkörper aus Draht hervorragend zur Verwendung als Knochenplatte eignen. Gegenüber herkömmlichen Knochenplatten aus Stahl oder Titan besitzen Knochenplatten aus einem gepressten Formkörper aus Draht eine gewisse Elastizität, die es zum einen ermöglicht, während der Operation kleinere Toleranzen, wie beispielsweise Krümmungen im Knochen oder nicht exakt gesetzte Bohrungen auszugleichen. Zum anderen ist ein gepresster Formkörper aus Draht gleichzeitig querelastisch, kann also leichten heilungsbedingten Verschiebungen der Knochenplatten zueinander folgen.

So ist das Einsetzen der erfindungsgemäßen Knochenplatten wesentlich einfacher, gleichzeitig kommt es nicht zu Verspannungen während des Heilungsprozesses.

Weiter kann ein gepresster Formkörper aus Draht eine sehr hohe Festigkeit besitzen. Insbesondere kann ein gepresster Formkörper aus Draht derart bereitgestellt werden, dass dessen Federkennlinie mit zunehmender Verformung ansteigt. Eine derartige Knochenplatte lässt zwar leichte Bewegungen und Verschiebungen zu, verhindert aber wirksam ein Auseinanderdriften der Knochenfragmente bei größeren Kräften.

Eine weiterer Vorteil einer erfindungsgemäßen Knochenplatte ist deren relativ geringes Gewicht im Verhältnis zu deren Festigkeit.

Vorzugsweise wird der gepresste Formkörper aus einer Maschenware ausgebildet. So wird zum einen die Festigkeit erhöht und zum anderen stehen keine Drahtspitzen aus dem Körper heraus.

Bei einer Weiterbildung der Erfindung ist die Maschenware als Gestrick, vorzugsweise als Rundgestrick ausgebildet. Beim Zusammenpressen verhaken sich die Maschen des Gestricks teilweise ineinander, wodurch ein Formkörper mit sehr hoher Festigkeit ausgebildet wird. Der Erfinder hat ferner herausgefunden, dass bei Verwendung eines Rundgestricks die Festigkeit des Formkörpers über dessen gesamtes Volumen besser ist. Bei nicht rundgestrickten Gestricken kann es möglicherweise zu Schwächungen des Körpers entlang der ehemaligen Ränder des als Flächengebilde ausgebildeten Gestricks kommen.

Vorzugsweise wird der gepresste Formkörper aus einer gefalteten oder aufgerollten Maschenware gebildet. So kann erreicht werden, dass der Draht beziehungsweise die Drahtmaschen gleichmäßig im Formkörper verteilt sind und dass sie sich gleichmäßig verteilt verhaken.

Bei einer Weiterbildung der Erfindung weist die Platte zumindest eine Aussparung zum Durchführen einer Schraube oder eines Drahtes auf, wobei die Rohdichte des Formkörpers im Bereich der Aussparung, also um die Aussparung herum, höher ist, als eines von der Aussparung beabstandeten Abschnitts des Formkörpers. Die Aussparung entspricht einer Bohrung bei einer herkömmlichen Knochenplatte. Das Einbringen von Bohrungen in einen gepressten Formkörper aus Draht würde aber die Struktur schädigen, da die Maschen in diesem Bereich zerstört werden. Daher werden die Aussparungen vorzugsweise beim Pressvorgang eingebracht, beispielsweise durch einen Stempel in der Form. Die Rohdichte des Formkörpers ist die Dichte der Knochenplatte, basierend auf deren Volumen einschließlich der Poren, wobei die Aussparungen nicht als Poren verstanden werden und dementsprechend das Volumen der Aussparungen nicht zum Volumen des Formkörpers gehört.

Eine erhöhte Rohdichte im Bereich der Aussparungen bedeutet, dass hier das Material dichter liegt, die Rohdichte somit erhöht ist. Die Festigkeit des Formkörpers wird so im Bereich der Aussparungen erhöht, was zum Einen den Materialverlust im Bereich der Aussparungen ausgleicht und zum Anderen für einen sichereren Halt von Verschraubungen sorgt. Bei herkömmlichen Knochenplatten aus massivem Material bedeuten Bohrungen immer Schwächungsstellen, in denen es insbesondere zu Rissbildung kommen kann.

Eine erhöhte Rohdichte im Bereich von Aussparungen kann bereits dadurch erreicht werden, dass eine Maschenware im Wesentlichen gleichmäßig verteilt in eine Pressform gegeben und zu einem Formkörper verpresst wird. Die Aussparungen werden dabei durch Stempel in der Form gebildet. Das von den Stempeln verdrängte Material erhöht die Rohdichte im Bereich der durch die Stempel geformten Aussparungen.

Vorzugsweise sind die Aussparungen randseitig abgeschrägt, so dass Schraubenköpfe in der Knochenplatte versenkt werden können. Auch die Herstellung von Abschrägungen ist bereits während des Pressprozesses, beispielsweise durch einen geeigneten Stempel, möglich.

Bei einer Weiterbildung der Erfindung umfasst der Formkörper zumindest zwei Abschnitte mit unterschiedlicher Rohdichte. Dabei ist insbesondere vorgesehen, einen ersten Abschnitt bereitzustellen, dessen Rohdichte zumindest 3%, vorzugsweise zumindest 10% und besonders bevorzugt zumindest 20% geringer ist, als die Rohdichte eines zweiten Abschnitts.

Es ist dabei nicht nur vorgesehen, die Rohdichte im Bereich von Aussparungen gegenüber der mittleren Rohdichte der Knochenplatte zu erhöhen, sondern gemäß der Erfindung ist auch vorgesehen, Bereiche mit reduzierter Rohdichte bereitzustellen. Derartige Bereiche können beispielsweise in einem mittleren Abschnitt des Formkörpers liegen, der dann eine höhere Elastizität besitzt und insbesondere kleineren Bewegungen der Knochenplatte während des Zusammenwachsens besser folgen kann.

Ein derartiger Formkörper kann beispielsweise dadurch bereitgestellt werden, indem eine Maschenware inhomogen verteilt in einer Form eingebracht wird, also im Bereich, in welchem der Formkörper eine verringerte Rohdichte haben soll, eine geringere Menge an Maschenware eingelegt wird.

Der Formkörper kann einen Porositätsgrad zwischen 20 und 80 %, vorzugsweise zwischen 30 und 70 % und besonders bevorzugt zwischen 40 und 60 % aufweisen.

Je nach Einsatzzweck können Platten mit einer Dicke zwischen 0,5 und 10 mm, vorzugsweise zwischen 1 und 8 mm und besonders bevorzugt zwischen 1,5 und 4 mm bereitgestellt werden.

Ein gepresster Formkörper aus einer Maschenware aus Draht ist somit nicht zwingend wesentlich dicker als herkömmliche Knochenplatten aus einem Massivmaterial.

Bei einer besonderen Ausführungsform der Erfindung besteht der Draht aus Titan oder einer Titanlegierung. Es hat sich gezeigt, dass Titan und Titanlegierungen besonders gut einwachsen. Die Verwendung von Titan bietet sich somit da an, wo eine besonders feste Verbindung der Knochenplatte mit dem umgebenden Gewebe indiziert ist.

Es ist aber auch denkbar, die Drähte des Formkörpers teilweise aus Titan und teilweise aus einem anderen Drahtmaterial zu formen, beispielsweise wenn ein Einwachsen zwar gewünscht ist, aber in reduzierter Form.

Als Material, welches nicht derart zum Einwachsen neigt wie Titan, ist insbesondere chirurgischer Edelstahl geeignet. Dieses Material besitzt in der Regel eine hohe Festigkeit und lässt sich gut zu einer Maschenware verarbeiten.

Bei einer alternativen Ausführungsform der Erfindung besteht der Draht aus einem bioresorbierbaren Material, insbesondere aus Magnesium oder einer Magnesiumlegierung. So lassen sich mit der Erfindung auch bioresorbierbare Knochenplatten bereitstellen, welche sich definiert abbauen.

Dem Abbauprozess kommt dabei zugute, dass ein gepresster Formkörper aus Draht eine hohe Porosität mit relativ großen, offenen Poren besitzt. Der Abbauprozess setzt daher nicht nur an den Außenseiten, sondern auch im Innern des Formkörpers an. Bei herkömmlichen massiven Knochenplatten würden Abbauprozesse von außen ansetzen. Dabei würde wahrscheinlich nach einiger Zeit eine derart hohe Zersetzung erreicht, dass im Bereich der Aussparungen kein sicherer Halt der Schrauben mehr möglich ist, die Knochenplatte also ihre Wirkung verliert. Der Abbauprozess des dann verbleibenden Restmaterials würde sich dann aber über einen viel längeren Zeitraum hinziehen.

Die Maschenweite kann 0,1 bis 50 mm, bevorzugt 0,5 bis 20 mm und besonders bevorzugt 3 bis 8 mm betragen.

Mit der Erfindung kann eine Platte bereitgestellt werden, welche zumindest abschnittsweise elastisch ist und eine anfängliche Federkonstante zwischen 50 und 3000 N/mm, bevorzugt zwischen 100 und 1000 N/mm und besonders bevorzugt zwischen 150 und 800 N/mm aufweist.

Gleichzeitig kann die Platte als eine Blattfeder betrachtet werden, welche bei randseitiger Befestigung und mittiger Auslenkung eine Federkonstante D zwischen 0,1 und 100 N/mm, bevorzugt zwischen 0,5 und 50 N/mm und besonders bevorzugt zwischen 1 und 20 N/mm haben kann.

Diese Federkonstante bezieht sich vorzugsweise auf die ersten 1 bis 2 mm Auslenkung, wobei die Federkonstante D mit zunehmender Auslenkung ansteigt, so dass die als Blattfeder zu betrachtende Knochenplatte kleineren Bewegungen folgen kann, bei größeren Bewegungen aber einen derart hohen Widerstand entgegensetzt, dass die Knochenfragmente sicher zusammen gehalten werden.

Die Erfindung betrifft des Weiteren ein Implantat, also nicht nur eine Knochenplatte, sondern beispielsweise auch ein Wirbelsäulenimplantat oder einen Teil einer Gelenkschale, welches einen gepressten Formkörper aus Draht umfasst, der eines oder mehrere der zuvor beschriebenen Merkmale aufweist, mit dem Unterschied, dass sich die jeweiligen Merkmale statt auf eine Knochenplatte auf ein Implantat im Allgemeinen beziehen. Gemäß der Erfindung ist der Draht zumindest teilweise beschichtet. Als Beschichtungen sind insbesondere Hartstoffbeschichtungen, Edelmetalle wie Gold oder Kunststoffbeschichtungen, insbesondere PTFE, vorgesehen. Durch eine derartige Beschichtung kann Abrieb bei Bewegungen, welche durch ein Aneinanderreiben der Drähte verursacht wird, zumindest reduziert werden.

Des Weiteren betrifft die Erfindung ein Implantat, welches einen gepressten Formkörper aus Draht umfasst, wobei der Draht zumindest teilweise aus einem bioresorbierbaren Material, insbesondere aus Magnesium oder einer Magnesiumlegierung besteht. So kann ein bioresorbierbares Material mit zuvor beschriebenem Vorteil auch für andere Einsatzgebiete verwendet werden.

Bei einer Weiterbildung der Erfindung ist der Draht mit einer bioresorbierbaren Beschichtung versehen. Insbesondere ist vorgesehen, einen Magnesiumdraht mit einer bioresorbierbaren Beschichtung zu umhüllen, wobei sich die bioresorbierbare Beschichtung nach einer vorbestimmten Zeit im Körper abbaut oder auflöst und dann den Magnesiumdraht zum Abbau freigibt. So können bioresorbierbare Implantate mit einer sehr genau definierten Einsatzzeit bereitgestellt werden, wobei das Material erst dann durch den Abbau geschwächt wird, wenn sich die Beschichtung abgelöst hat, sodann der Abbau des Implantats aber relativ schnell erfolgt.

Die Erfindung betrifft des Weiteren ein Verfahren zur Herstellung einer Platte zur Fixierung von Knochenfragmenten. Dabei wird eine Maschenware gefaltet und/oder aufgerollt und sodann die Maschenware in einer Presse zu einem Formkörper verpresst. Unter einem Formkörper wird dabei ein Körper verstanden, dessen Form im Wesentlichen durch die Form der Presse vorgegeben wird, wobei klar ist, dass nach dem Pressen sich der Körper etwas entspannt und somit nicht genau der Form der Presse annimmt.

Vorzugsweise wird eine rundgestrickte Maschenware verwendet.

Bei einer Weiterbildung der Erfindung wird die Maschenware derart gefaltet und/oder aufgerollt, dass in zumindest einem Bereich weniger Lagen übereinander liegen als in einem weiteren Bereich.

So kann ein Formkörper bereitgestellt werden, welcher über das Volumen eine variierende Rohdichte hat, insbesondere kann ein Formkörper bereitgestellt werden, bei welchem die Schwächungszone im Wesentlichen mittig angeordnet ist. Unter mittig im Sinne der Erfindung wird ein Bereich verstanden, welcher sich zwischen den Befestigungsaussparungen der Knochenplatte befindet.

Um die Aussparungen zu formen, wird vorzugsweise eine Pressform mit einem Stempel verwendet. Dabei kann beispielsweise ein Stift in die Form eingesteckt werden. Bei größeren Platten ist es sogar möglich, mit einer einzigen Form Knochenplatten bereitzustellen, die an unterschiedlichen Stellen Aussparungen aufweisen. Dabei können die Stifte an unterschiedlichen Positionen der Form eingesteckt werden.

Vorzugsweise wird eine geprägte Maschenware verwendet. Insbesondere kann eine Maschenware verwendet werden, welche eine durch Prägen gewellte Oberfläche hat.

Die Verwendung einer geprägten Maschenware erhöht die Tendenz der Maschen, sich beim Pressvorgang miteinander zu verhaken, wodurch ein Formkörper mit höherer Festigkeit bereitgestellt werden kann.

Der Formkörper wird vorzugsweise mit einem hydraulischen oder pneumatischen Presswerkzeug geformt. Gegenüber beispielsweise Exzenter-Pressen haben diese Werkzeuge den Vorteil einer in der Regel relativ langsamen Verformung, insbesondere beim Abschluss des Pressvorgangs. So haben beim Pressvorgang die Maschen mehr Zeit, sich miteinander zu verhaken.

Die Maschenware kann, wie es bei einer Weiterbildung der Erfindung vorgesehen ist, auch zunächst gefaltet und sodann aufgerollt werden.

### Kurzbeschreibung der Zeichnungen

Die Erfindung soll im Folgenden bezugnehmend auf die Zeichnungen Fig. 1 bis Fig. 4 näher erläutert werden.
Fig. 1 zeigt schematisch eine beispielhafte Knochenplatte,
bezugnehmend auf Fig. 2 soll das Falten einer Maschenware näher erläutert werden,
bezugnehmend auf Fig. 3 soll die Wirkung der Knochenplatte als Blattfeder näher erläutert werden,
Fig. 4 zeigt schematisch ein Flussdiagramm mit den wesentlichen Verfahrensschritten zum Herstellen einer Knochenplatte nach einem Ausführungsbeispiel der Erfindung.

### Detaillierte Beschreibung der Zeichnungen

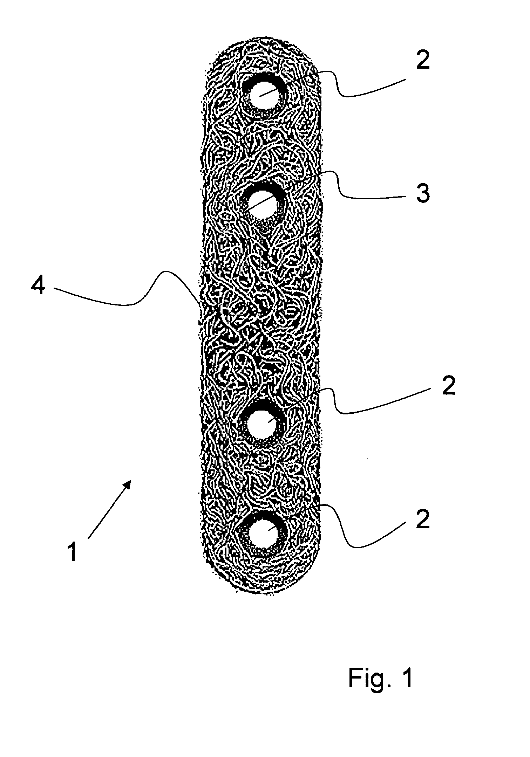

Fig. 1 zeigt eine schematisch dargestellte Platte 1 zur Fixierung von Knochenfragmenten, im Folgenden auch Knochenplatte genannt. Die Platte 1 besteht aus einem Gestrick aus Draht und besitzt in diesem Ausführungsbeispiel vier Aussparungen 2. Die Aussparungen 2 wurden mittels Stempeln beim Pressvorgang eingebracht und besitzen eine Abschrägung 3 zum Einsenken einer Schraube (nicht dargestellt).

Zu erkennen ist, dass die Drähte um die Aussparung herum dichter liegen, mithin also die Rohdichte im Bereich der Aussparungen 2 höher ist.

Bei dieser Knochenplatte wurde zusätzlich im mittleren Bereich 4 das Gestrick lockerer gelegt, und zwar dadurch, dass weniger Lagen übereinander gelegt wurden. Somit ist im mittleren Bereich 4 die Rohdichte nochmals reduziert, was zu einer erhöhten Elastizität im mittleren Bereich 4 führt.

Die Knochenplatte ist in diesem Ausführungsbeispiel als einfache gerade Lasche zum Verbinden zweier Knochenfragmente ausgebildet.

Es versteht sich, dass die Knochenplatte für andere Einsatzzwecke beliebige andere Formen aufweisen kann.

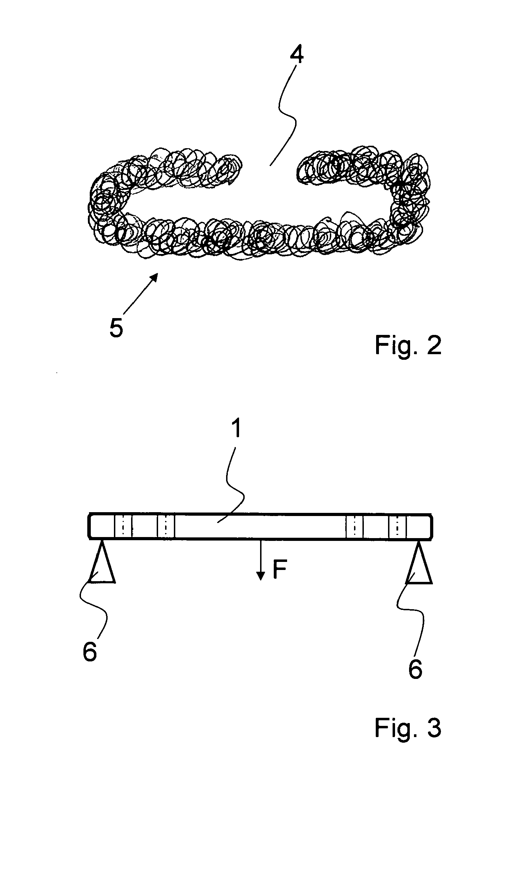

Bezug nehmend auf Fig. 2 soll ein wesentlicher Schritt im Herstellungsvorgang einer Knochenplatte näher erläutert werden.

Zu erkennen ist schematisch dargestellt ein Gestrick 5, welches in diesem Beispiel einmal gefaltet wird. Im mittleren Bereich 4 wird das Gestrick aber derart gefaltet, dass keine zwei Lagen übereinander liegen.

Nach Einlegen des Gestricks 5 in eine Form (nicht dargestellt), kann so eine Knochenplatte bereitgestellt werden, deren Rohrdichte im mittleren Bereich 4 reduziert ist.

Bezugnehmend auf Fig. 3 soll die Betrachtung einer Knochenplatte 1 als Blattfeder näher erläutert werden.

Die Knochenplatte 1 ist randseitig auf den Lagern 6 gelagert. Mittig wird mit einer Kraft F an der Knochenplatte senkrecht nach unten gezogen und die Auslenkung gemessen. Die anfängliche Federkonstante D liegt dabei zwischen 0,5 und 50 N/mm, nimmt aber mit zunehmender Auslenkung zu, so dass die Knochenplatte trotz ihrer Elastizität eine sichere Fixierung der Knochenplatten bei größeren Kräften ermöglicht.

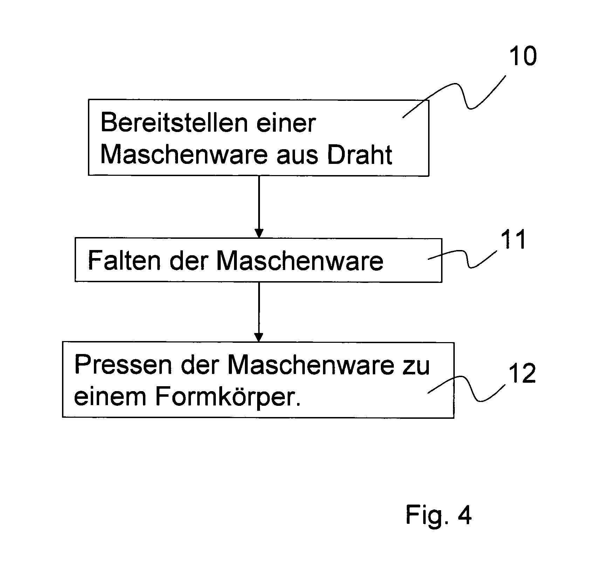

Bezug nehmend auf Fig. 4 sollen die wesentlichen Schritte eines Herstellungsverfahrens erläutert werden.

Zunächst wird eine Maschenware aus Draht bereitgestellt 10. Die Maschenware wird sodann gefaltet 11. Alternativ oder zusätzlich kann die Maschenware auch aufgerollt werden.

Sodann wird die Maschenware zu einem Formkörper verpresst 12. Die Form des Formkörpers wird dabei im Wesentlichen durch die Form der Presse vorgegeben.

Es versteht sich, dass die Erfindung nicht auf eine Kombination vorstehend beschriebener Merkmale beschränkt ist, sondern dass der Fachmann sämtliche Merkmale, soweit dies sinnvoll ist, kombinieren wird.

### Bezugszeichenliste

- 1: Platte
- 2: Bohrung
- 3: Abschrägung
- 4: mittlerer Bereich
- 5: Gestrick
- 6: Lager
- 10: Bereitstellen einer Maschenware aus Draht
- 11: Falten der Maschenware
- 12: Pressen der Maschenware zu einem Formkörper

## Patentansprüche

1. Platte zur Fixierung von Knochenfragmenten, **dadurch gekennzeichnet, dass** die Platte einen gepressten Formkörper aus Draht umfasst, insbesondere aus einem gepressten Formkörper aus Draht besteht.

2. Platte zur Fixierung von Knochenfragmenten nach dem vorstehenden Anspruch, **dadurch gekennzeichnet, dass** der gepresste Formkörper aus einer Maschenware ausgebildet ist.

3. Platte zur Fixierung von Knochenfragmenten nach dem vorstehenden Anspruch, **dadurch gekennzeichnet, dass** die Maschenware als Gestrick ausgebildet ist.

4. Platte zur Fixierung von Knochenfragmenten nach einem der vorstehenden Ansprüche, **dadurch gekennzeichnet, dass** die Platte zumindest eine Aussparung zum Durchführen einer Schraube oder eines Drahtes aufweist, wobei die Rohdichte des Formkörpers im Bereich der Aussparung höher ist, als eines von der Aussparung beabstandeten Abschnitts des Formkörpers.

5. Platte zur Fixierung von Knochenfragmenten nach einem der vorstehenden Ansprüche, **dadurch gekennzeichnet, dass** die Platte zumindest eine randseitig abgeschrägte Aussparung aufweist.

6. Platte zur Fixierung von Knochenfragmenten nach einem der vorstehenden Ansprüche, **dadurch gekennzeichnet, dass** die Rohdichte des Formkörpers in Richtung eines mittleren Bereiches hin abnimmt.

7. Platte zur Fixierung von Knochenfragmenten nach einem der vorstehenden Ansprüche, **dadurch gekennzeichnet, dass** die Platte zumindest eine Aussparung aufweist, wobei die Rohdichte eines die Aussparung umgebenden Bereiches höher ist als eines weiteren von der Aussparung entfernten Bereiches.

8. Platte zur Fixierung von Knochenfragmenten nach einem der vorstehenden Ansprüche, **dadurch gekennzeichnet, dass** die Platte zumindest abschnittsweise elastisch ist und eine Federkonstante anfänglich zwischen 50 und 3000 N/mm, bevorzugt zwischen 100 und 1000 N/mm, besonders bevorzugt zwischen 150 und 800 N/mm beträgt.

9. Implantat, insbesondere mit einem oder mehreren Merkmalen nach einem der vorstehenden Ansprüche, welches einen gepressten Formkörper aus Draht umfasst, **dadurch gekennzeichnet, dass** der Draht zumindest teilweise beschichtet ist.

10. Implantat, insbesondere mit einem oder mehreren Merkmalen nach einem der vorstehenden Ansprüche, welches einen gepressten Formkörper aus Draht umfasst, **dadurch gekennzeichnet, dass** der Draht zumindest teilweise aus Magnesium oder einer Magnesiumlegierung besteht.

11. Implantat nach dem vorstehenden Anspruch, **dadurch gekennzeichnet, dass** der Draht mit einer bioresorbierbaren Beschichtung versehen ist.

12. Verfahren zur Herstellung einer Platte zur Fixierung von Knochenfragmenten, insbesondere einer Platte nach einem der vorstehenden Ansprüche, umfassend die Schritte:
- Bereitstellen einer Maschenware aus Draht,
- Falten und/oder Aufrollen der Maschenware,
- Pressen der Maschenware zu einem Formkörper.

13. Verfahren zur Herstellung einer Platte zur Fixierung von Knochenfragmenten nach dem vorstehenden Anspruch, **dadurch gekennzeichnet, dass** eine rundgestrickte Maschenware verwendet wird.

14. Verfahren zur Herstellung einer Platte zur Fixierung von Knochenfragmenten nach einem der vorstehenden Ansprüche, **dadurch gekennzeichnet, dass** die Maschenware derart gefaltet und/oder aufgerollt wird, dass in zumindest einem Bereich weniger Lagen übereinander angeordnet sind als in einem weiteren Bereich.

15. Verfahren zur Herstellung einer Platte zur Fixierung von Knochenfragmenten nach einem der vorstehenden Ansprüche**, dadurch gekennzeichnet, dass** eine Pressform mit zumindest einem Stempel verwendet wird, um zumindest eine Aussparung in der Platte auszubilden.
